(19) Europäisches Patentamt

European Patent Office

Office européen des brevets

(11) **EP 1 639 989 B1**

(12) **EUROPEAN PATENT SPECIFICATION**

(45) Date of publication and mention
of the grant of the patent:
**09.11.2011 Bulletin 2011/45**

(51) Int Cl.:
*A61K 8/86* (2006.01)          *A61K 8/33* (2006.01)

(21) Application number: **05020527.7**

(22) Date of filing: **21.09.2005**

(54) **Microemulsion**

Mikroemulsion

Microémulsion

(84) Designated Contracting States:
**DE FR GB**

(30) Priority: **22.09.2004 JP 2004274971**

(43) Date of publication of application:
**29.03.2006 Bulletin 2006/13**

(73) Proprietor: **KAO CORPORATION**
**Tokyo 103-8210 (JP)**

(72) Inventor: **Tomokuni, Atsushi**
**Sumida-ku**
**Tokyo 131-8501 (JP)**

(74) Representative: **HOFFMANN EITLE**
**Patent- und Rechtsanwälte**
**Arabellastraße 4**
**81925 München (DE)**

(56) References cited:
**WO-A-2005/004835     WO-A-2005/063193**
**US-A- 5 575 990**

## Description

### Field of the Invention

The present invention relates to a microemulsion.

### Background of the Invention

To obtain a microemulsion which is a liquid composition containing an oily component sollubilized in an aqueous phase or an aqueous component liquidized in an oil phase, surfactants, such as nonionic surfactants have been used in many cases. However, since polyethylene glycol-based nonionic surfactants such as polyethylene glycol alkyl ethers generally undergo a certain change in their affinity for water as the temperature rises, a microemulsion using such surfactants will have a very narrow temperature range of its stability from the critical solubilization temperature to the cloud point in the phase diagram.

Broadly, there are three methods known for improving the temperature stability of microemulsion even in the use of nonionic surfactants.

The first known method includes using a nonionic surfactant in combination with an anionic surfactant In "Solution and Solubility" written by Kouzo Shinoda, p.177, published by Maruzen Co., Ltd., Tokyo, is disclosed a composition providing a microemulsion using an isocapryl alcohol monoglyceryl ether of a nonionic surfactant and a sodium palmitylsulfonate salt of an anionic surfactant Further, J. Phys. Chem., 92, 4702 (1988), JP-A-58-128311 and JP-A-58-131127 also disclose such methods as combining a nonionic surfactant with an anionic surfactant.

As stated above, it has been said that a combination of a specific nonionic surfactant and anionic surfactant is effective for maintaining the resultant microemulsion in its stable state even if the temperature changes. However, since this method requires the use of a specific nonionic surfactant and anionic surfactant at a specific formulation ratio, a problem will arise in that the degree of flexibility in formulation is inevitably limited when trying to apply such a microemulsion to cosmetic articles or like preparations.

A second method to improve the temperature stability of a microemulsion while using a nonionic surfactant includes controlling the composition of the aqueous phase or oil phase used.

JP-A-6-293617 discloses a composition providing a microemulsion, the composition using a nonionic surfactant as a surfactant and containing polar solvent as an aqueous component, but not containing water. This method is stated as being able to expand the temperature range in which a microemulsion can be stable. However, with such a composition without a water content, neither a fresh feeling of use nor a feeling of hydration upon use which is to no small extent desirable for cosmetic articles can be achieved.

JP-A-10-43573 discloses a composition providing a microemulsion, containing as a nonionic surfactant a sucrose fatty acid ester, having a specific ester distribution, of a saturated or unsaturated fatty acid having a carbon number of 12 to 22 and, containing a monohydric alcohol having a carbon number of 4 to 20. This method is also stated as being able to expand the temperature range in which a microemulsion can be stable. In this method, however, if a monohydric alcohol having a smaller carbon number is used, its distinctive odor will result in a microemulsion-based cosmetic article having a degraded aroma, while if a monohydric alcohol having a larger carbon number is used, an oiliness of the alcohol will render it difficult to produce a feeling of use desired for cosmetic articles.

JP-A-63-126543 and JP-A-63-126544 describe a microemulsion containing a hydrophilic nonionic surfactant, an oil having its inorganic value and carbon number limited to a specific range on an organic conceptual diagram, and water. However, the microemulsions disclosed by those patent applications are hardly applicable to cosmetic articles, because the type of oil, the surfactant content and the mixing ratio of oil and water are limited to a specific range.

A third method to improve the temperature stability of a microemulsion while using a nonionic surfactant includes controlling the type and/or combination of nonionic surfactants used.

JP-A-6-262060 discloses a microemulsion sucrose fatty acid ester, alkylglucoside or polyethylene glycol containing, as essential ingredients, a sucrose fatty acid ester, an alkylglucoside or polyethylene glycol, oily component, and water, wherein said sucrose fatty acid ester and said alkylglucoside or polyethylene glycol are used in such a combination that one of these ingredients exists as hydrophilic and the other as lipophilic. However, the temperature span in which the compositions disclosed there remain stable is at most on the order of 26 ˚C, and thus the compositions do not have a sufficient temperature span allowing their practical use as cosmetic materials. Besides, the sucrose fatty acid ester represents a surfactant relatively liable to hydrolysis among nonionic surfactants and thus is not necessarily preferred for cosmetic articles in view of stability in long term storage.

JP-A-11-262653 discloses an oil-in-water type microemulsion containing polyglycerin fatty acid ester, a reaction product of a polyglycerin having an average degree of polymerization ranging from 5 to 15 and a fatty acid having 75 wt% or higher pure oleic acid content, and a polyhydric alcohol. However, this oil-in-water type microemulsion is not preferred for application to cosmetic articles, because it employs limitedly specified nonionic surfactants leading to low versatility and because in most cases the polyglyceryl fatty acid ester uses, as its fatty acid, oleic acid which is a typical unsaturated fatty acid involving a stability problem such as coloring or separation with time when stored for a longer period.

In Journal of Oleo Science, Vol.51, No.6, 379-386(2002), is described a method for producing a microemulsion using a sucrose fatty acid ester as the above-described oily component, water and nonionic surfactant However, for the microemulsion described there, no such compositions are available that are stable at around room temperatures where the microemulsion is used as cosmetic articles.

Thus, the well-known methods of the prior art to produce a temperature-stable composition for a microemulsion using a nonionic surfactant have not been successful satisfiable for applications to cosmetic articles in respect of ingredient composition, feeling of use preferable for cosmetic articles, or stability of the resultant cosmetics on skins, etc. Further, even if any microemulsions obtained by the prior art methods are applicable to cosmetic articles, such emulsions have not been able to exist stably over a sufficiently wide temperature range.

Summary of the invention

The present invention provides a microemulsion, containing the following ingredients (A) through (F):

(A) a hydrophilic nonionic surfactant having as a hydrophilic group a residue of a sugar, reducing sugar or polyglycerin having a hydrogen atom of at least one hydroxyl group thereof removed;
(B) a hydrophilic nonionic surfactant having a polyoxyethylene chain as a hydrophilic group;
(C) a water-soluble organic solvent selected from the group consisting of (C1) through (C3) listed below;

(C1) a compound having in a molecule thereof two or more oxypropylene groups (PO) and hydroxyl groups (OH), the ratio in number of these two groups (PO/OH) being smaller than 5;
(C2) a monohydric alcohol having a carbon number ranging from 2 to 6; and
(C3) a dihydric alcohol having a carbon number ranging from 2 to 6;

(D) a lipophilic nonionic surfactant as defined in claim 1;
(E) an oily ingredient; and
(F) water.

Further, the present invention provides a skin cosmetic and a skin cleansing composition which contain the microemulsion according to the present invention.

Detailed Description of the invention

The present invention provides a microemulsion having a stability over a wider temperature range.

The inventors have found out that a certain combination of three specific surfactants with specific water-soluble organic solvents can produce a microemulsion which is stable over a wider temperature range.

The microemulsion according to the present invention has an excellent stability over a wide temperature range.

The "microemulsion" may be defined in two ways, namely, in a broader sense and in a narrower sense. That is to say, there are one case ("microemulsion in the narrow sense") in which the microemulsion refers to a thermodynamically stable isotropic single liquid phase containing a ternary system having three ingredients of an oily component, an aqueous component and a surfactant, and the other case ("microemulsion in the broad sense") in which among thermodynamically unstable typical emulsion systems the microemulsion additionally includes those such emulsions presenting transparent or translucent appearances due to their smaller particle sizes (Satoshi Tomomasa, et al., Oil Chemistry, Vol. 37, No. 11 (1988), pp. 48-53). The "microemulsion" as used herein refers to a "microemulsion in the narrow sense," i.e., a thermodynamically stable isotropic single liquid phase.

The microemulsion refers to either one state of an O/W (oil-in-water type microemulsion in which oil is solubilized by micelles, a W/O (water-in-oil) type microemulsion in which water is solubilized by reverse micelles, or a bicontinuous microemulsion in which the number of associations of surfactant molecules are rendered infinite so that both the aqueous phase and oil phase have a continuous structure.

For properties, the microemulsion appears transparent or translucent and may exist as a solution in a monophasic state in which all the formulated ingredients and components are uniformly dissolved therein.

Regardless of manufacturing processes, microemulsions may take the same state if they have the same formulation and same temperature. Therefore, the above-described three ingredients and the remaining ingredients may be added and mixed in any orders as appropriate and may be agitated using mechanical forces at any power to consequently yield a microemulsion having substantially the same state.

Thus, whether a composition containing the above-described three ingredients is a microemulsion or not may be determined based on its properties and manufacturing process. In respect of properties, the microemulsion takes a liquid state at room temperatures (about 25 ˚C) and whether a composition constitute a microemulsion may be determined by measuring its viscosity. For example, 10,000 mPa·s or lower viscosity (a viscometer manufactured by TOKIMEC INC., Tokyo, measurement conditions: rotor No. 1, 60 rpm) may be employed as a measure of the microemulsion state. For the manufacturing process, whether at least two processes involving varied mixing orders and mixing temperatures of ingredients yield the same state (in appearance, viscosity, feeling of use, etc.) at the same temperature may be depended on to determine the existence of a microemulsion state.

According to the present invention, the hydrophilic nonionic surfactant as the above-described ingredient (A) has as a hydrophilic group a residue of a sugar, reducing sugar or polyglycerin having a hydrogen atom of at least one hydroxyl group thereof removed and includes, for example, polyglyceryl fatty acid esters, polyglyceryl alkyl ethers, sucrose fatty acid esters, alkylpolyglucosides, or the like.

For the present microemulsion, preferable polyglyceryl fatty acid esters are those esters of a polyglycerin and a fatty acid having a carbon number ranging from 8 to 22, including, for example, polyglyceryl octanoate esters, polyglyceryl 2-ethylhexylate esters, polyglyceryl decanoate esters, polyglyceryl laurate esters, polyglyceryl myristate esters, polyglyceryl palmitate esters, polyglyceryl isostearate esters, polyglyceryl stearate esters, polyglyceryl oleate esters, polyglyceryl behenate esters, and so on. Among these esters, monoesters of a polyglycerin having a degree of polymerization ranging from 3 to 15 and a fatty acid having a carbon number ranging from 12 to 18 are more preferred.

Preferable polyglyceryl alkyl ethers are those ethers of a polyglycerin and an alkyl group having a carbon number ranging from 8 to 22, including, polyglyceryl octyl ethers, polyglyceryl decyl ethers, polyglyceryl lauryl ethers, polyglyceryl myristyl ethers, polyglyceryl palmityl ethers, polyglyceryl isostearyl ethers, polyglyceryl stearyl ethers, polyglyceryl oleyl ethers, polyglyceryl behenyl ethers, and so on. Among these ethers, monoethers of a polyglycerin having a degree of polymerization ranging from 3 to 15 and an alkyl group having a carbon number ranging from 12 to 18 are more preferred.

Preferable sucrose fatty acid esters are those esters derived from a fatty acid having a carbon number ranging from 8 to 22 and sucrose, including, for example, sucrose octanoate esters, sucrose 2-ethylhexanoate esters, sucrose decanoate esters, sucrose laurate esters, sucrose myristate esters, sucrose palmitate esters, sucrose isostearate esters, sucrose stearate esters, sucrose oleate esters, sucrose behenate esters, and so on. Among these, monoesters of a fatty acid having a carbon number ranging from 12 to 18 and sucrose are more preferred.

Preferable alkylpolyglucosides are those having an alkyl group with a carbon number ranging from 8 to 22 and a degree of glucoside unit condensation ranging from 1 to 7, including, for example, octylpolyglucosides, 2-ethylhexylpolyglucosides, decylpolyglucosides, laurylpolyglucosides, myristylpolyglucosides, palmitylpolyglucosides, isostearylpolyglucosides, stearyl laurylpolyglucosides, oleylpolyglucosides, behenylpolyglucosides, and so on. More preferably, such alkylpolyglucosides as having an alkyl group with a carbon number ranging from 8 to 11 and having a degree of glucoside unit condensation ranging from 1 to 1.4 may be used and, further preferably, those having an alkyl group with a carbon number of 12 to 14 and degree of glucoside unit condensation ranging from 1.5 to 4.0 may be used.

For the hydrophilic nonionic surfactant as the above-described ingredient (A), it is more preferable to use polyglyceryl fatty acid esters, polyglyceryl alkyl ethers or alkylpolyglucosides, because the resultant composition may obtain a high stability in long term storage.

The ingredient (A) may contain one or more such hydrophilic nonionic surfactants with its content ranging preferably from 0.05 to 8 weight % and more preferably from 0.1 to 7 weight % of the microemulsion, because such a content allows the microemulsion to have a stability over a wider temperature range around the room temperatures and a good feeling of use as a cosmetic article.

According to the present invention, the hydrophilic nonionic surfactant as the ingredient (B) has a polyoxyethylene chain as a hydrophilic group and includes, for example, polyoxyethylene fatty acid esters, polyoxyethylene alkyl ethers, polyoxyethylene sorbitan fatty acid esters, polyoxyethylene glycerin monofatty acid esters, polyoxyethylene hydrogenated castor oils, polyoxyethylene hydrogenated castor oil monofatty acid esters, and so on.

Preferable polyoxyethylene fatty acid esters are those esters derived from a fatty acid with a carbon number ranging from 8 to 22 and having a degree of polymerization of ethylene oxide (hereinafter, shall be abbreviated as "EO") ranging from 5 to 60, including, polyoxyethylene octanoate esters, polyoxyethylene 2-ethylhexanoate esters, polyoxyethylene decanoate esters, polyoxyethylene laurate esters, polyoxyethylene myristate esters, polyoxyethylene palmitate esters, polyoxyethylene isostearate esters, polyoxyethylene stearate esters,polyoxyethylene oleate esters, polyoxyethylene behenate esters, and so on. Among these, esters derived from a fatty acid with a carbon number ranging from 12 to 18 and having a degree of EO polymerization ranging from 5 to 60 are more preferred.

Preferable polyoxyethylene alkyl ethers are those ethers having an alkyl group with a carbon number ranging from 8 to 22 and having a degree of EO polymerization ranging from 5 to 60, including polyoxyethylene octyl ethers, polyoxyethylene decyl ethers, polyoxyethylene lauryl ethers, polyoxyethylene myristyl ethers, polyoxyethylene palmityl ethers, polyoxyethylene isostearyl ethers, polyoxyethylene stearyl ethers, polyoxyethylene oleyl ethers, polyoxyethylene behenyl ethers, and so on. Among these, ethers having an alkyl group with a carbon number ranging from 12 to 18 and a degree of EO polymerization ranging from 5 to 60 are more preferred.

Preferable polyoxyethylene sorbitan fatty acid esters are those esters derived from a fatty acid with a carbon number ranging from 8 to 22 and having degree of EO polymerization ranging from 5 to 60 and include, for example, polyoxyethylene sorbitan octanoate esters, polyoxyethylene sorbitan decanoate esters, polyoxyethylene sorbitan laurate esters, polyoxyethylene sorbitan myristate esters, polyoxyethylene sorbitan palmitate esters, polyoxyethylene sorbitan isostearate esters, polyoxyethylene sorbitan stearate esters, polyoxyethylene sorbitan oleate esters, polyoxyethylene behenate esters, and so on. Among these, esters derived from a fatty acid with a carbon number ranging from 12 to 18 and having a degree of EO polymerization ranging from 5 to 60 are more preferred.

Preferable polyoxyethylene glycerin monofatty acid esters are those esters derived from a fatty acid with a carbon number ranging from 8 to 22 and having degree of EO polymerization ranging from 5 to 60, including polyoxyethylene glycerin monooctanoate esters, polyoxyethylene glycerin monodecanoate esters, polyoxyethylene glycerin monolaurate esters, polyoxyethylene glycerin monomyristate esters, polyoxyethylene glycerin monoisostearate esters, polyoxyethylene glycerin monostearate esters, polyoxyethylene glycerin monooleate esters, polyoxyethylene glycerin monobehenate esters, and so on. Among these, esters derived from a fatty acid with a carbon number ranging from 12 to 18 and having a degree of EO polymerization ranging from 5 to 60 are more preferred.

Preferable polyoxyethylene hydrogenated castor oils are those having a degree of EO polymerization ranging from 20 to 80 and more preferable from 30 to 60.

Meanwhile, preferable polyoxyethylene hydrogenated castor oil monofatty acid esters are those esters derived from a fatty acid with a carbon number ranging from 8 to 22 and having a degree of EO polymerization ranging from 20 to 80 and include, for example, polyoxyethylene hydrogenated castor oil monooctanoate esters, polyoxyethylene hydrogenated castor oil monodecanoate esters, polyoxyethylene hydrogenated castor oil monolaurate esters, polyoxyethylene hydrogenated castor oil monomyristate esters, polyoxyethylene hydrogenated castor oil monopalmitate esters, polyoxyethylene hydrogenated castor oil monoisostearate esters, polyoxyethylene hydrogenated castor oil monostearate esters, polyoxyethylene hydrogenated castor oil monooleate esters, polyoxyethylene hydrogenated castor oil monobehenate esters, and so on. Among these, esters derived from a fatty acid with a carbon number ranging from 12 to 18 and having a degree of EO polymerization ranging from 20 to 80 are more preferred.

For the hydrophilic nonionic surfactant as the above-described ingredient (B), it is more preferable to use polyoxyethylene fatty acid esters, polyoxyethylene alkyl ethers, polyoxyethylene sorbitan fatty acid esters, or polyoxyethylene glycerin monofatty acid esters, because the resultant composition may obtain a good feeling of use as a cosmetic article.

The ingredient (B) may contain one or more such nonionic surfactants, and its content ranges preferably from 0.05 to 8 weight % and more preferably from 0.1 to 7 weight % of the microemulsion, because such a content allows the resultant microemulsion to have a stability over a wider temperature range around the room temperatures and a good feeling of use as a cosmetic article.

Further, for the ingredients (A) and (B), it is preferred to use hydrophilic nonionic surfactants having an HLB (hydrophile-lypophile balance) value above 8 and more preferably above 9. Here, the HLB value represents a measure of the molecular weight shared by the hydroxyl group portion of a surfactant in its total molecular weight and for polyoxyethylene-based nonionic surfactants it can be determined by the Griffin's formula shown below.

$$\text{HLB value} = E/5$$

where: E represents the quantity in weight % of the polyoxyethylene part contained in a surfactant molecule.

According to the present invention, the ratio (A)/(B) in weight of the ingredients (A) and (B) ranges preferably from 0.1 to 10 and more preferably from 0.2 to 5, because within this range the resultant microemulsion can have a stability over a wider temperature range around the room temperatures.

According to the present invention, the water-soluble organic solvent as the aforementioned ingredient (C) is selected from the group consisting of (C1) through (C3) listed below:

(C1) a compound having in a molecule thereof two or more oxypropylene groups (PO) and hydroxyl groups (OH), the ratio in number of these two groups (PO/OH) being smaller than 5;
(C2) a monohydric alcohol having a carbon number ranging from 2 to 6; and
(C3) a dihydric alcohol having a carbon number ranging from 2 to 6.

The polypropylene glycol-based compound, namely the foregoing compound (C1) preferably has a PO/OH ratio smaller than or equal to 4. Further, it is preferred that the above compound (C) have a PO/OH ratio ranging from 1.5 to 4, because such a range allows the resultant microemulsion to have a stability over a wider temperature range around the room temperatures and a good feeling of use as a cosmetic article.

Preferable polypropylene glycol-based compounds include polypropylene glycols, polyoxypropylene trimethylpropane ethers, polyoxypropylene sorbitol ethers, polyoxypropylene monoglyceryl ethers, polyoxypropylene diglyceryl ethers, polyoxypropylene triglyceryl ethers, monoalkyl ethers having a polyoxypropylene polyglyceryl ether skeleton, condensates of a polypropylene glycol and a polyglucoside, condensates of a polypropylene glycol and sucrose, and so on.

Among these, polypropylene glycols, polyoxypropylene trimethylpropane ethers, polyoxypropylene sorbitol ethers, and compounds having a propylene oxide structure of a monoglycerin and/or a polyglycerin are preferred and, further specifically, polypropylene glycols, polyoxypropylene trimethylpropane ethers, polyoxypropylene sorbitol ethers, polyoxypropylene monoglyceryl ethers, polyoxypropylene diglyceryl ethers, polyoxypropylene triglyceryl ethers are more preferred.

Meanwhile, it is preferred to use a monohydric alcohol having a carbon number of 2 or 3 and a dihydric alcohol having a carbon number ranging from 2 to 6 as the components (C2) and (C3), respectively, selectable for the ingredient (C).

Such monohydric and dihydric alcohols include ethanol, propanol, isopropanol, butanol, propylene glycol, isoprene glycol, 1,3-butylene glycol, 1,2-pentanediol, 1,2-hexanediol, hexylene glycol, etc. and, among these, ethanol, propylene glycol, isoprene glycol, 1,3-butylene glycol and hexylene glycol are more preferred.

The ingredient (C) may contain one or more such compounds as described above, and its content ranges preferably from 0.5 to 35 weight % and more preferably from 1.0 to 30 weight % of the microemulsion, because such a content allows the microemulsion to have a stability over a wider temperature range around the room temperatures and a good feeling of use as a cosmetic article.

According to the present invention, the lipophilic nonionic surfactant as the aforementioned ingredient (D) preferably has an HLB value not greater than 8. Here, the HLB value can be - determined using the Griffin's formula described previously. Specifically, such surfactants are selected from polyoxyethylene difatty acid esters, polyoxyethylene dialkyl ethers, monoglycerin monofatty acid esters, as defined in claim 1, monoglycerin difatty acid esters, diglycerin monofatty acid esters, monoglyceryl monoalkyl ethers, diglyceryl monoalkyl ethers, sorbitan fatty acid esters.

More specifically, for the polyoxyethylene difatty acid esters, it is preferred to use diesters derived from a fatty add having a carbon number ranging from 8 to 22 and a polyethylene glycol having a degree of EO polymerization ranging from 2 to 14, including, for example, polyoxyethylene octanoate esters, polyoxyethylene 2-ethyhexanoate esters, polyoxyethylene decanoate esters, polyoxyethylene laurate esters, polyoxyethylene myristate esters, polyoxyethylene palmitate esters, polyoxyethylene isosterate esters, polyoxyethylene stearate esters, polyoxyethylene oleate esters, polyoxyethylene behenate esters, and so on. Among these, esters derived from a fatty acid with a carbon number ranging from 12 to 18 and having a degree of EO polymerization ranging from 4 to 12 are more preferred.

For the polyoxyethylene dialkyl ethers, it is preferred to use diethers having an alkyl group with a carbon number ranging from 8 to 22 and having a degree of EO polymerization ranging from 2 to 14, including polyoxyethylene octyl ethers, polyoxyethylene decyl ethers, polyoxyethylene lauryl ethers, polyoxyethylene myristyl ethers, polyoxyethylene palmityl ethers, polyoxyethylene isostearyl ethers, polyoxyethylene stearyl ethers, polyoxyethylene oleyl ethers, polyoxyethylene behenyl ethers, and so on. Among these, ethers having an alkyl group with a carbon number ranging from 12 to 18 and a degree of EO polymerization ranging from 4 to 12 are more preferred.

For the monoglycerin difatty acid esters, it is preferred to use diesters derived from glycerin and a fatty acid having a carbon number ranging from 8 to 22, including, monoglycerin octanoate ester, monoglycerin 2-ethylhexanoate ester, monoglycerin decanoate ester, monoglycerin laurate ester, monoglycerin myristate ester, monoglycerin palmitate ester, monoglycerin oleate ester, or monoglycerin behenate ester. Among these, monoglycerin 2-ethylhexanoate ester, monoglycerin laurate ester, monoglycerin myristate ester, monoglycerin palmitate ester, and monoglycerin oleate ester are more preferred.

For the diglycerin monofatty acid ester, it is preferred to use those ester derived from a diglycerin and a fatty acid having a carbon number ranging from 8 to 22, including, diglycerin octanoate ester, diglycerin 2-ethylhexanoate ester, diglycerin caprylate ester, diglycerin caprate ester, diglycerin laurate ester, diglycerin myristate ester, diglycerin palmitate ester, diglycerin isostearate ester, diglycerin stearate ester, diglycerin oleate ester, diglycerin behenate ester, and so on. Among these, diglycerin 2-ethylhexanoate ester, diglycerin laurate ester, diglycerin myristate ester, diglycerin palmitate ester, diglycerin isostearate ester and diglycerin stearate ester are more preferred.

Preferable monoglyceryl monoalkyl ethers are those ethers of glycerin and an alkyl group having a carbon number ranging from 8 to 22, including, monoglyceryl 2-ethylhexyl ether, monoglyceryl octyl ether, monoglyceryl decyl ether, monoglyceryl lauryl ether, monoglyceryl myristyl ether, monoglyceryl palmityl ether, monoglyceryl stearyl ether, monoglyceryl isostearyl ether, monoglyceryl oleyl ether, monoglyceryl behenyl ether, and so on. Among these, monoglyceryl 2-ethylhexyl ether, monoglyceryl lauryl ether, monoglyceryl myristyl ether, monoglyceryl palmityl ether, monoglyceryl stearyl ether and monoglyceryl isostearyl ether are more preferred.

Preferable diglyceryl monoalkyl ethers are those ethers derived from diglycerin and an alkyl group having a carbon number ranging from 8 to 22, including diglyceryl 2-ethylhexyl ether, diglyceryl octyl ether, diglyceryl decyl ether, diglyceryl lauryl ether, diglyceryl myristyl ether, diglyceryl palmityl ether, diglyceryl stearyl ether, diglyceryl isostearyl ether, diglyceryl oleyl ether, diglyceryl behenyl ether, and so on. Among these, driglyceryl 2-ethylhexyl ether, diglyceryl lauryl ether, diglyceryl myristyl ether, diglyceryl palmityl ether, diglyceryl stearyl ether and diglyceryl isostearyl ether are more preferred.

Preferable sorbitan fatty acid ester are those ester derived from a fatty acid having a carbon number ranging from 8 to 22, including, sorbitan octanoate ester, sorbitan 2-ethylhexanoate ester, sorbitan caprylate ester, sorbitan caprate ester, sorbitan laurate ester, sorbitan myristate ester, sorbitan palmitate ester, sorbitan isostearate ester, sorbitan stearate ester, sorbitan oleate ester, sorbitan behenate ester, and so on. Among these, sorbitan 2-ethylhexanoate ester, sorbitan laurate ester, sorbitan myristate ester, sorbitan palmitate ester, sorbitan isostearate ester and sorbitan stearate ester are more preferred.

For the ingredient (D), diglycerin monofatty acid esters, monoglycerin monofatty acid esters as defined in claim 1, monoglycerin difatty acid esters, monoglyceryl monoalkyl ethers, diglyceryl monoalkyl ethers and sorbitan fatty acid esters are more preferred.

The ingredient (D) may contain one or more such compounds as described above, and its content ranges preferably from 0.05 to 8 weight % and more preferably from 0.1 to 7 weight % of the microemulsion, because such a content allows the resultant microemulsion to have a stability over a wider temperature range around the room temperatures and a good feeling of use as a cosmetic article.

According to the present invention, it is preferred that the total content (A) + (B) + (D) of the foregoing nonionic surfactant ingredients (A), (B) and (D) range from 02 to 10 weight % and more preferably from 0.4 to 9 weight % of the entire composition, because such a content allows the resultant microemulsion to have a stability over a wider temperature range around the room temperatures and a good feeling of use as a cosmetic article.

According to the present invention, preferable oily components as the aforementioned ingredient (E) may be those materials ordinarily used for cosmetic articles, including, for example, hydrocarbon oils such as liquid paraffin, liquid isoparaffin, squalane, etc.; ester oils such as cholesteryl isostearate, isopropyl palmitate, isopropyl myristate, neopentylglycol dicaprate, isopropyl isostearate, octadecyl myristate, cetyl 2-ethylhexanoate, isononyl isononanoate, isotridecyl isononanoate, glyceryl tri-2-ethylhexanoate, glyceryl tri(caprylate/caprate), etc.; ether oils such as alkyl-1,3-dimethylethyl ether, nonylphenyl ether, etc.; silicone oils such as methyl polysiloxane, decamethylcyclopentasiloxane, octamethylcyclotetrasiloxane, etc.; higher fatty acids having a carbon number ranging from 8 to 22, such as lauric acid, myristic acid, palmitic acid, stearic acid, etc.; higher alcohols having a carbon number ranging from 8 to 22, such as lauryl alcohol, myristyl alcohol, palmityl alcohol, stearyl alcohol, cetyl alcohol, etc.; animal and vegetable oils such as fish oil, soybean oil, olive oil, etc.; ceramides, phospholipids, glycolipids, etc.; or terpene oils.

Among these, liquid paraffin, liquid isoparaffin, neopentylglycol dicaprate, isopropyl isostearate, cetyl 2-ethylhesanoate, isononyl isononanoate, glyceryl tri(caprylate/caprate), alkyl-1,3-dimethylbutyl ether, methyl polysiloxane having a molecular weight ranging from 100 to 500, decamethylcyclopentasiloxane, octamethylcyclotetrasiloxane, higher fatty acids having a carbon number ranging from 12 to 22, higher alcohols having a carbon number ranging from 12 to 22, soybean oil, olive oil, ceramides, glycolipids and terpene oil are more preferred and, further, hydrocarbon oils are also preferred.

The ingredient (E) may contain one or more such compounds as described above, and its content ranges preferably from 1 to 50 weight % and more preferably from 2 to 45 weight % of the microemulsion, because such a content allows the microemulsion to have a stability over a wider temperature range around the room temperatures and a good feeling of use as a cosmetic article.

Further, it is preferred that the present microemulsion contains the above-described ingredient (F), namely water, in a quantity ranging from 40 to 95 weight % and more preferably from 45 to 90 weight %, because such a content allows the microemulsion to have a stability over a wider temperature range around the room temperatures and a good feeling of use as a cosmetic article.

The microemulsion of the present invention may be mixed as appropriate with such additional ingredients that are typically used for cosmetic articles, including any surfactants or water-soluble solvents other than those specified hereinbefore, thickeners, bactericides, humectants, wetting agents, colorants, preservatives, feel improving agents, perfumes, antiinflammatory agent, skin-lightening cosmetics, antihidrotics, ultraviolet absorbers, etc.

The microemulsion of the present invention may be produced by any typical methods known in the art and may be applied in the form of skin cleansing composition such as cleanser, face wash, body wash, etc.; skin cosmetic such as skin toning lotion, essence, skin-lightening cosmetic, antiwrinkling agent, anti-UV skin care cosmetic, etc. Further, the present microemulsion may be applied in the form of such cosmetic as combined with woven textiles, nonwoven fabrics or like sheet materials.

Examples

Preferred examples 1 through 16 and comparative examples 1 through 6
Microemulsions having the formulations shown in Tables 1 through 3 were prepared to evaluate the temperature stabilities. Tables also show the results of evaluation.

Process

For preparation of the microemulsions, the process basically proceeded in the following way. All ingredients of each intended composition was loaded into a mixing vessel as a batch. To dissolve those normally solid components or those gelled components produced by mixing at room temperatures, the batch was heated at 70 to 75 ˚C under agitation. After the content was fully dissolved, the batch was cooled down to room temperature to obtain a microemulsion. It is to be noted here that neither the order and manner of mixing the ingredients nor the speed of their agitation is not particularly limited according to the present invention. Also, the heating temperature is not limited to those temperatures mentioned above.

Evaluation method

For each microemulsion prepared as above, five 20 mt samples were separately put into a capped dear glass vessel and held at 5 ˚C, 10 ˚C, 25 ˚C, 40 ˚C and 45 ˚C, respectively, for 12 hours. Then, the microemulsion samples were externally observed visually and rated as good "o" when the samples were uniform without turbidity and their viscosity was sufficiently low, while rating as bad "x" when the samples underwent separation.
[Table 1]

| Ingredients (weight %) | | | Preferred examples | | | | | | | |
|---|---|---|---|---|---|---|---|---|---|---|
| | | | 1 | 2 | 3 | 4 | 5 | 6 | 7 | 8 |
| A | Polyglyceryl (5) monostearate ester | Sunsoft A-181E(by Taiyo Kagaku Co., Ltd., Mie, JP), HLB=13 | | 2.00 | 2.00 | | | 2.00 | 2.00 | 3.00 |
| | Sucrose laurate ester | Surfhope SE COSMEC-1216 (by Mitublshi-Kagaku Foods Corp., Tokyo), HLB=16 | | | | 1.00 | | | | |
| | Alkylglycoside | Mydol 10 (by Kao Corp., Tokyo), HLB=17 | | | | | 3.75 | | | |
| | Polyglyceryl (5) monoisostearate ester | Sunsoft A-19E (by Taiyo Kagaku Co., Ltd. Mie, JP), HLB=13 | 2.00 | | | | | | | |
| | Polyglyceryl (5) laurate ester | Sunsoft A-121E (by Taiyo Kagaku Co., Ltd., Mie, JP), HLB=12 | | | | | | | | |
| | Sucrose stearate ester | Surfhope SE COSMEC-1816 (by Mitubishi-Kagaku Foods Corp., Tokyo), HLB=16 | | | | | | | | |
| B | POE (12) laurate ester | Emanon 1112 (by Kao Corp., Tokyo),HLB=13.3 | | | 3.20 | | | | | 3.00 |
| | POE(20)sorbitan laurate | Rheodol TW-L120 (by Kao Corp, Tokyo), HLB=16.7 | 2.25 | 2.80 | | 2.20 | 2.20 | 2.60 | 2.60 | |
| 1 C | PPG (9) diglyceryl ether | SY-DP9 (by Sakamoto Yakuhin Kogyo Co., Ltd., Osaka, JP), PO/OH=2.3 | 5.50 | 10.00 | 10.00 | 10.00 | 10.00 | | | |
| | Dipropylene glycol | ADEKA DPG-RF (by Asahidennka Co., Ltd., Tokyo), PO/OH=1.0 | | | | | | 15.00 | | |
| | PPG(3.4) | Newpol PP-200 (by Sanyo Chemical Industries, Ltd. Kyoto, JP), PO/OH=1.7 | | | | | | | 15.00 | |
| | PPG (8.8) monoglyceryl ether | Newpol PP-600 (by Sanyo Chemical Industries, Ltd, Kyoto, JP), PO/OH=2.9 | | | | | | | | 13.00 |
| | PPG (14) diglyceryl) ether | SY-DP14 (by Sakamoto Yakuhin Kogyo Co., Ltd, Osaka, JP), PO/OH=3.5 | | | | | | | | |
| | Ethanol | | | | | | | | | |
| | Propylene glycol | | | | | | | | | |
| D | Isostearylglyceryl ether | Penetol GE-IS(by Kao Corp., Tokyo). HLB=5.3 | 1.75 | 1.20 | 0.80 | 1.80 | 1.80 | 1.40 | 1.40, | 1.00 |

(continued)

| Ingredients (weight %) | | | Preferred examples | | | | | | | |
|---|---|---|---|---|---|---|---|---|---|---|
| | | | 1 | 2 | 3 | 4 | 5 | 6 | 7 | 8 |
| E | Hydrogenated polyisobutene | | 44.25 | 10.00 | 5.00 | 10.00 | 10.00 | 10.00 | 10.00 | 10.00 |
| F | Water | | 44.25 | 74.00 | 79.00 | 75.00 | 72.25 | 69.00 | 69.00 | 70.00 |
| | Disodium hydrogenphosphate | | | | | | | | | |
| | Sodium hydrogen phosphate | | | | | | | | | |
| | Carrageenan | Soagina MV-101 (Mitsubishi Rayon Co., Ltd.) | | | | | | | | |
| Stability at 5˚C | | | ○ | ○ | ○ | ○ | ○ | ○ | ○ | ○ |
| 10˚C | | | ○ | ○ | ○ | ○ | ○ | ○ | ○ | ○ |
| 25˚C | | | ○ | ○ | ○ | ○ | ○ | ○ | ○ | ○ |
| 40˚C | | | ○ | ○ | ○ | ○ | ○ | ○ | ○ | ○ |
| 45˚C | | | ○ | ○ | ○ | ○ | ○ | ○ | ○ | ○ |

[Table 2]

| Ingredients (weight %) | | | Examples | | | | | | | |
|---|---|---|---|---|---|---|---|---|---|---|
| | | | 9 | 10 | 11 | 12 | 13 | 14 | 15 | 16 |
| A | Polyglyceryl (5) monostearate ester | Sunsoft A-181E (by Taiyo Kagaku Co., Ltd. Mie, JP), HI8=13 | 2.00 | | | | | | 2.00 | 2.00 |
| | Sucrose laurate ester | Surfhope SE COSMEC-1216 (by Mitubishi-Kagaku Foods Corp., Tokyo). HLB=16 | | | | | 2.50 | | | |
| | Alkylglucoside | Mydol 10 (by Kao Corp., Tokyo), HLB=17 | | | | | | | | |
| | Polyglyceryl (5) monoisostearate ester | Sunsoft A-19E(by Taiyo Kagaku Co., Ltd. Mie, JP), HLB=13 | | 2.00 | | 2.00 | | | | |
| | Polyglyceryl (5) laurate ester | Sunsoft A-121E (by Taiyo Kagaku Co., Ltd. Mie, JP), HLB=12 | | | 2.00 | | | | | |
| | Sucrose stearate ester | Surfhope SE COSMEC-1816 (by Mitubishi-Kagaku Foods Corp., Tokyo), HLB=16 | | | | | | 2.00 | | |
| B | POE (12) laurate ester | Emanon 1112 (by Kao Corp., Tokyo) HLB=13.3 | | 2.60 | 3.20 | 2.60 | 0.80 | 2.00 | 2.89 | 2.89 |
| | POE (20) sorbitan laurate | Rheodol TW-L120 (by Kao Corp, Tokyo), HLB=16.7 | 2.60 | | | | | | | |
| C | PPG (9) diglyceryl ether | SY-DP9 (by Sakamoto Yakuhin Kogyo Co., Ltd., Osaka, Japan), PO/OH=2.3 | | | | | 5.00 | 7.50 | 10.00 | 10.00 |
| | Dipropylene glycol | ADEKA DPG-RF (by Asahidennka Co., Ltd., Tokyo), PO/OH=1.0 | | | | | | | | |
| | PPG(3.4) | Newpol PP-200 (by Sanyo Chemical Industries, Ltd., Kyoto, JP), PO/OH=1.7 | | | | | | | | |
| | PPG (8.8) monoglyceryl ether | Newpol PP-600 (by Sanyo Chemical Industries, Ltd., Kyoto, JP), PO/OH=2.9 | | | | | | | | |
| | PPG (14) diglyceryl ether | SY-DP14 (by Sakamoto Yakuhin Kogyo Co., Ltd., Osaka, JP), PO/OH=3.5 | 10.00 | | | | | | | |
| | Ethanol | | | 25.00 | 20.00 | | | 5.00 | 5.00 | 5.00 |
| | Propylene glycol | | | | | 25.00 | | | | |
| D | Isostearylglyceryl ether | Penetol GE-IS (by Kao Corp., Tokyo), HLB=5.3 | 1.40 | 1.40 | 0.80 | 1.40 | 1.70 | 1.00 | 1.11 | 1.11 |

(continued)

| Ingredients (weight %) | | | Examples | | | | | | | |
|---|---|---|---|---|---|---|---|---|---|---|
| | | | 9 | 10 | 11 | 12 | 13 | 14 | 15 | 16 |
| E | Hydrogenated polyisobutene | | 10.00 | 5.00 | 5.00 | 5.00 | 15.00 | 15.00 | 15.00 | 15.00 |
| F | Water | | 74.00 | 64.00 | 69.00 | 64.00 | 74.50 | 67.00 | 64.00 | 63.50 |
| | Disodium hydrogenphosphate | | | | | | 0.35 | 0.35 | | |
| | Sodium hydrogenphosphate | | | | | | 0.15 | 0.15 | | |
| | Carrageenan | Soagina MV-101 (by Mitsubishi Rayon Co., Ltd.) | | | | | | | | 0.50 |
| Stability at 5˚C | | | ○ | ○ | ○ | ○ | ○ | ○ | ○ | ○ |
| 10˚C | | | ○ | ○ | ○ | ○ | ○ | ○ | ○ | ○ |
| 25˚C | | | ○ | ○ | ○ | ○ | ○ | ○ | ○ | ○ |
| 40˚C | | | ○ | ○ | ○ | ○ | ○ | ○ | ○ | ○ |
| 45˚C | | | ○ | ○ | ○ | ○ | ○ | ○ | ○ | ○ |

[Table 3]

| Ingredients (weight%) | | | Comparative examples | | | | | |
|---|---|---|---|---|---|---|---|---|
| | | | 1 | 2 | 3 | 4 | 5 | 6 |
| A | Potyglyceyl(5) monostearate ester | Sunsoft A-181E (by Taiyo Kagaku Co., Ltd. Mie, JP), HLB=13 | | 2.00 | 2.00 | 2.00 | 2.00 | 2.00 |
| B | POE (12) laurate ester | Emanon 1112 (by Kao Corp., Tokyo 3.70 HLB=13.3 | | | | | 2.60 | 2.60 |
| | POE (20) sorbitan laurate | Rheodol TW-L120 (by Kao Corp, Tokyo), HLB=16.7 | | | 4.00 | 3.00 | | |
| C | PPG(9) diglyceryl ether | SY-DP9 (by Sakamoto Yakuhin Kogyo Co., Ltd., Osaka, Japan), PO/OH=23 | 10.00 | 10.00 | | 10.00 | | |
| | PPG(10) | Newpol PP-600 (by Sanyo Chemical Industries, Ltd., Kyoto, JP), PO/OH=5.0 | | | | | 9.00 | |
| | PPG(24) diglyceryl ether | PO/OH=6.0 | | | | | | 8.00 |
| D | Isostearylglyceryl ether | Penetol GE-IS (by Kao Corp., Tokyo), HLB=5.3 | 1.30 | 1.40 | 1.00 | | 1.40 | 1.40 |
| E | Hydrogenated polyisobutene | | 15.00 | 10.00 | 10.00 | 15.00 | 10.00 | 10.00 |
| F | Water | | 70.00 | 76.60 | 83.00 | 70.00 | 75.00 | 76.00 |
| Stability at 5°C | | | × | × | ○ | × | ○ | ○ |
| 10°C | | | × | × | ○ | × | ○ | × |
| 25°C | | | ○ | × | ○ | × | ○ | × |
| 40°C | | | × | ○ | × | × | × | × |
| 45°C | | | × | ○ | × | × | × | × |

**Claims**

1. A microemulsion comprising the following ingredients (A) through (F):

(A) a hydrophilic nonionic surfactant having as a hydrophilic group a residue of a sugar, reducing sugar or polyglycerin having a hydrogen atom of at least one hydroxyl group thereof removed;
(B) a hydrophilic nonionic surfactant having a polyoxyethylene chain as a hydrophilic group;
(C) a water-soluble organic solvent selected from the group consisting of (C1) through (C3) listed below;

(C1) a compound having in a molecule thereof two or more oxypropylene groups (PO) and hydroxyl groups (OH) the ratio in number of said two groups (PO/OH) being smaller than 5;
(C2) a monohydric alcohol having a carbon number ranging from 2 to 6; and
(C3) a dihydric alcohol having a carbon number ranging from 2 to 6;

(D) a lipophilic nonionic surfactant selected from the group consisting of polyoxyethylene difatty acid esters, polyoxyethylene dialkyl ethers, monoglycerin monofatty acid esters, monoglycerin difatty acid esters, diglycerin monofatty acid esters, monoglyceryl monoalkyl ethers, diglyceryl monoalkyl ethers and sorbitan fatty acid esters, wherein the monoglycerin monofatty acid esters are selected from monoglycerin 2-ethylhexanoate ester, monoglycerin laurate ester, monoglycerin myristate ester, monoglycerin palmitate ester and monoglycerin oleate ester;
(E) an oily ingredient; and
(F) water.

2. The microemulsion according to claim 1, wherein said hydrophilic nonionic surfactant constituting said ingredient (A) is selected from the group consisting of polyglyceryl fatty acid esters, polyglyceryl alkyl ethers, sucrose fatty acid esters and alkylpolyglucosides.

3. The microemulsion according to claim 1 or 2, wherein said hydrophilic nonionic surfactant constituting said ingredient (B) is selected from the group consisting of polyoxyethylene fatty acid esters, polyoxyethylene alkyl ethers, polyoxyethylene sorbitan fatty acid esters, polyoxyethylene glycerin monofatty acid esters, polyoxyethylene hydrogenated castor oils and polyoxyethylene hydrogenated castor oil monofatty acid esters.

4. The microemulsion according to any one of claims 1 through 3, wherein said compound in said ingredient (C) having in a molecule thereof two or more oxypropylene groups (PO) and hydroxyl groups (OH) with the ratio in number of said two groups (PO/OH) being smaller than 5 is selected from the group consisting of polypropylene glycol, propylene oxide adducts of monoglycerin and propylene oxide adducts of polyglycerin.

5. The microemulsion according to any one of claims 1 through 3, wherein said monohydric alcohol or said dihydric alcohol having a carbon number ranging from 2 to 6 in said ingredient (C) is selected from the group consisting of ethanol, propylene glycol, isoprene glycol, 1,3-butylene glycol and hexylene glycol.

6. The microemulsion according to any one of claims 1 through 5, wherein the ratio (A)/(B) in weight of said ingredients (A) and (B) ranges from 0.1 to 10.

7. A skin cosmetic comprising a microemulsion according to any one of claims 1 through 6.

8. A skin cleansing composition comprising a microemulsion according to any one of the preceding claims 1 through 6.

**Patentansprüche**

1. Mikroemulsion, umfassend die folgenden Bestandteile (A) bis (F):

(A) ein hydrophiles nicht-ionisches Tensid, das als hydrophile Gruppe einen Rest eines Zuckers, reduzierenden Zuckers oder Polyglycerins aufweist, wobei ein Wasserstoffatom von zumindest einer Hydroxylgruppe davon entfernt ist;
(B) ein hydrophiles nicht-ionisches Tensid mit einer Polyoxyethylenkette als hydrophile Gruppe;
(C) ein wasserlösliches organisches Lösungsmittel, ausgewählt aus der Gruppe, bestehend aus (C1) bis (C3), wie unten angegeben;

(C1) eine Verbindung, die in einem Molekül davon 2 oder mehrere Oxypropylengruppen (PO) und Hydroxylgruppen (OH) aufweist, wobei das Zahlenverhältnis der beiden Gruppen (PO/OH) kleiner als 5 ist;
(C2) einen 1-wertigen Alkohol mit einer Kohlenstoffzahl im Bereich von 2 bis 6; und
(C3) einen 2-wertigen Alkohol mit einer Kohlenstoffzahl von 2 bis 6;

(D) ein lipophiles nicht-ionisches Tensid, ausgewählt aus der Gruppe, bestehend aus Polyoxyethylen-Difettsäureestern, Polyoxyethylen-Dialkylethern, Monoglycerin-Monofettsäureestern, Monoglycerin-Difettsäureestern, Diglycerin-Monofettsäureestern, Monoglyceryl-Monoalkylethern, Diglyceryl-Monoalkylether und Sorbitanfettsäureestern, worin die Monoglycerin-Monofettsäureester ausgewählt sind aus Monoglycerin-2-ethylhexanoatester, Monoglycerinlauratester, Monoglycerinmyristatester, Monoglycerinpalmitatester und Monoglycerinoleatester;
(E) einen öligen Bestandteil; und
(F) Wasser.

2. Mikroemulsion nach Anspruch 1, worin das hydrophile nicht-ionische Tensid, das den Bestandteil (A) ausmacht, ausgewählt ist aus der Gruppe, bestehend aus Polyglyceryl-Fettsäureestern, Polyglyceryl-Alkylethern, Succrosefettsäureestern und Alkylpolyglycosiden.

3. Mikroemulsion nach Anspruch 1 oder 2, worin das hydrophile nicht-ionische Tensid, das den Bestandteil (B) ausmacht, ausgewählt ist aus der Gruppe, bestehend aus Polyoxyethylen-Fettsäureestern, Polyoxyethylen-Alkylethern, Polyoxyethylen-Sorbitanfettsäureestern, Polyoxyethylen-Glycerinmonofettsäureestern, Polyoxyethylen-hydrierten Castorölen und Polyoxyethylen-hydrierten Castoröl-Monofettsäureestern.

4. Mikroemulsion nach einem der Ansprüche 1 bis 3, worin die Verbindung in dem Bestandteil (C), die in einem Molekül davon 2 oder mehr Oxypropylengruppen (PO) und Hydroxylgruppen (OH) aufweist, wobei das Zahlenverhältnis der zwei Gruppen (PO/OH) kleiner als 5 ist, ausgewählt ist aus der Gruppe bestehend aus Polypropylenglycol, Propylenoxidaddukten von Monoglycerin und Propylenoxidaddukten von Polyglycerin.

5. Mikroemulsion nach einem der Ansprüche 1 bis 3, worin der 1-wertige Alkohol oder der 2-wertige Alkohol mit einer Kohlenstoffzahl im Bereich von 2 bis 6 im Bestandteil (C) ausgewählt ist aus der Gruppe bestehend aus Ethanol, Propylenglycol, Isoprenglycol, 1,3-Butylenglycol und Hexylenglycol.

6. Mikroemulsion nach einem der Ansprüche 1 bis 5, worin das Gewichtsverhältnis (A)/(B) der Bestandteile (A) und (B) im Bereich von 0,1 bis 10 liegt.

7. Hautkosmetikum, umfassend eine Mikroemulsion nach einem der Ansprüche 1 bis 6.

8. Hautreinigungszusammensetzung, umfassend eine Mikroemulsion nach einem der vorhergehenden Ansprüche 1 bis 6.

**Revendications**

1. Microémulsion comprenant les ingrédients (A) à (F) suivants :

   (A) un tensioactif non ionique hydrophile ayant comme groupe hydrophile, un résidu de sucre, de sucre réducteur ou de polyglycérine ayant un atome d'hydrogène d'au moins un groupe hydroxyle retiré de celui-ci ;
   (B) un tensioactif non ionique hydrophile ayant une chaîne de polyoxyéthylène comme groupe hydrophile ;
   (C) un solvant organique hydrosoluble choisi dans le groupe constitué des composés (C1) à (C3) énumérés ci-dessous ;

   (C1) un composé ayant dans une molécule de celui-ci, deux groupes oxypropylène (OP) et groupes hydroxyle (OH), ou plus, le rapport en nombre desdits deux groupes (PO/OH) étant inférieur à 5 ;
   (C2) un alcool monohydrique ayant un nombre d'atomes de carbone de 2 à 6 ; et
   (C3) un alcool dihydrique ayant un nombre d'atomes de carbone de 2 à 6 ;

   (D) un tensioactif non ionique lipophile choisi dans le groupe constitué des diesters d'acides gras de polyoxyéthylène, des dialkyléthers de polyoxyéthylène, des monoesters d'acides gras de monoglycérine, des diesters d'acides gras de monoglycérine, des monoesters d'acides gras de diglycérine, des monoalkyléthers de monoglycéryle, des monoalkyléthers de diglycéryle et des esters d'acides gras de sorbitane, les monoesters d'acides gras de monoglycérine étant choisis parmi le 2-éthylhexanoate de monoglycérine, l'ester de laurate de monoglycérine, l'ester de myristate de monoglycérine, l'ester de palmitate de monoglycérine et l'ester d'oléate de monoglycérine ;
   (E) un ingrédient huileux ; et
   (F) de l'eau

2. Microémulsion selon la revendication 1, dans laquelle ledit tensioactif non ionique hydrophile constituant ledit ingrédient (A) est choisi dans le groupe constitué des esters d'acides gras de polyglycéryle, des alkyléthers de polyglycéryle, des esters d'acide gras de saccharose et des alkylpolyglucosides.

3. Microémulsion selon la revendication 1 ou 2, dans laquelle ledit tensioactif non ionique hydrophile constituant ledit ingrédient (B) est choisi dans le groupe constitué des esters d'acides gras de polyoxyéthylène, des alkyléthers de polyoxyéthylène, des esters d'acides gras de polyoxyéthylène-sorbitane, des monoesters d'acides gras de polyoxyéthylène-glycérine,des huiles de ricin hydrogénées de polyoxyéthylène et des monoesters d'acides gras d'huile de ricin hydrogénée de polyoxyéthylène.

4. Microémulsion selon l'une quelconque des revendications 1 à 3, dans laquelle ledit composé dans ledit ingrédient (C) ayant dans une molécule de celui-ci, deux groupes oxypropylène (PO) et groupes hydroxyle (OH) ou plus, le rapport en nombre desdits deux groupes (PO/OH) étant inférieur à 5, est choisi dans le groupe constitué du polypropylène glycol, des adduits d'oxydes de propylène de monoglycérine et des adduits d'oxyde de propylène de polyglycérine.

5. Microémulsion selon l'une quelconque des revendications 1 à 3, dans laquelle ledit alcool monohydrique ou ledit alcool dihydrique ayant un nombre d'atomes de carbone de 2 à 6 dans ledit ingrédient (C) est choisi dans le groupe constitué de l'éthanol, du propylène glycol, de l'isoprène-glycol, du 1,3-butylène glycol et de l'hexylène glycol.

6. Microémulsion selon l'une quelconque des revendications 1 à 5, dans laquelle le rapport (A)/(B) en poids desdits ingrédients (A) et (B) est de 0,1 à 10.

7. Produit cosmétique cutané comprenant une microémulsion selon l'une quelconque des revendications 1 à 6.

8. Composition de nettoyage de la peau comprenant une microémulsion selon l'une quelconque des revendications 1 à 6 précédentes.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 58128311 A
- JP 58131127 A
- JP 6293617 A
- JP 10043573 A

- JP 63126543 A
- JP 63126544 A
- JP 6262060 A
- JP 11262653 A

**Non-patent literature cited in the description**

- **Kouzo Shinoda.** Solution and Solubility. Maruzen Co., Ltd, 177
- *J. Phys. Chem.,* 1988, vol. 92, 4702

- *Journal of Oleo Science,* 2002, vol. 51 (6), 379-386
- **Satoshi Tomomasa et al.** *Oil Chemistry,* 1988, vol. 37 (11), 48-53